# EUROPÄISCHE PATENTSCHRIFT

(11) **EP 2 120 896 B1**
(45) Veröffentlichungstag und Bekanntmachung des Hinweises auf die Patenterteilung: **24.05.2017**
(21) Anmeldenummer: 08700515.3
(22) Anmeldetag: 10.01.2008
(51) Int. Cl.: A61K 9/70, A61K 31/245

(54) **MEDIZINISCH WIRKSAMES PFLASTER**
MEDICALLY ACTIVE PLASTER
PANSEMENT À ACTION MÉDICALE

(30) Priorität: 11.01.2007 CH 33072007; 31.05.2007 CH 871072007
(43) Veröffentlichungstag der Anmeldung: 25.11.2009
(73) Patentinhaber: Drossapharm Ag, 4144 Arlesheim (CH)
(72) Erfinder: IMBODEN, Roger, CH-4142 Münchenstein (CH); LUTZ, Jürg, CH-4102 Binningen (CH)
(74) Vertreter: Braun, André jr.
(86) Internationale Anmeldenummer: PCT/CH2008/000010
(87) Internationale Veröffentlichungsnummer: WO 2008/083508

(56) Entgegenhaltungen:
- EP-A2- 0 150 426
- WO-A-2006/064576
- US-A- 4 623 346
- US-A- 5 306 503
- US-A- 5 830 505
- US-A- 5 902 601
- US-A1- 2001 053 383
- US-A1- 2006 263 419
- US-B1- 6 316 022

## Beschreibung

Die vorliegende Erfindung betrifft ein medizinisch wirksames Pflaster für die Abgabe von bei Raumtemperatur flüssigen pharmazeutischen Wirkstoffen an die Haut, insbesondere für die Abgabe des antiphlogistisch wirkenden Wirkstoffs Etofenamat.

Medizinisch wirksame Pflaster für die Abgabe von pharmazeutischen Wirkstoffen an die Haut sind an sich bekannt. Diese Pflaster bestehen in der Regel aus einer Deckschicht, einer Trägerschicht enthaltend den pharmazeutischen Wirkstoff, und einer abziehbaren Schutzschicht. Die Herstellung solcher Pflaster ist jedoch schwierig, denn die mit der Haut in Kontakt stehende Trägerschicht muss den optimalen Übergang des Wirkstoffs in die Haut gewährleisten und gleichzeitig auf der Haut weniger fest haften als auf der Decksicht, damit das Pflaster ohne weiteres und vollständig von der Haut entfernt werden kann.

Dabei soll die Trägerschicht eine möglichst hohe Konzentration des Wirkstoffs enthalten, damit dieser über eine vergleichsweise lange Zeitdauer in einer pharmazeutisch wirksamen Konzentration vom Pflaster abgegeben werden kann. Dies ist für bei Raumtemperatur flüssige Wirkstoffe schwierig zu erreichen, da flüssige Wirkstoffe in der Trägerschicht stabil eingelagert und mit dem Material der Trägerschicht kompatibel sein müssen. Auch muss der Wirkstoff vom Trägermaterial in genügender Geschwindigkeit über eine längere Zeitdauer abgegeben werden. Dabei spielt nicht nur die Art des Trägermaterials sondern auch die chemische Struktur des Wirkstoffs eine wichtige Rolle.

Aus US 6,316,022 ist ein medizinisch wirksames Pflaster bekannt, in welchem die den Wirkstoff enthaltende Trägerschicht aus einer Polyacrylatverbindung besteht und der Wirkstoff in der Trägerschicht gelöst ist. US 5 830 505 beschreibt ein medizinisch wirksames Pflaster, in welchem als Trägermaterial ein Acrylatcopolymer verwendet wird, in welchem der Wirkstoff gelöst ist. US 2001/053383 betrifft ein medizinisches Pflaster, in welchem die Trägerschicht aus einer Polyacrylat/Polysiloxan-Mischung besteht und der Wirkstoff in der Matrix gelöst ist. US 2006/263419 betrifft ein medizinisches Pflaster, in welchem die Trägerschicht beispielsweise Polyvinylpyrrolidon, ein Copolymer von Vinylpyrrolidon und Vinylacetat oder ein Copolymer von Äthylen und Vinylacetat und den Wirkstoff als feste Partikel enthält. WO 2006/064576 beschreibt ein Pflaster, worin die Trägerschicht eine Mischung bestehend aus einem nicht-klebenden synthetischen Gummi, einem klebenden Harz und einem flüssigen Paraffin, darstellt. US 4,623,346 beschreibt ein analoges Pflaster, worin die Trägerschicht aus mehreren Komponenten besteht, ausgewählt aus einem synthetischen Polymer, einem_Fliesshilfsmittel und einem klebenden Harz.

Es wurde nun gefunden, dass sich der allgemein schlecht lösliche antiphlogistisch wirkende Wirkstoff Etofenamat überraschenderweise in reiner und gleichmässig fein verteilter Form stabil in eine selbstklebende Silikonmatrix einlagern lässt und darin eine fein verteilte Dispersion bildet, so dass eine Matrix mit sehr guten Klebeeigenschaften erhalten wird, welche den Wirkstoff über eine längere Zeitdauer in wirksamer Konzentration abgibt und erfindungsgemäss als Trägerschicht verwendet werden kann.

Die vorliegende Erfindung ist in den Patentansprüchen definiert. Die vorliegende Erfindung betrifft ein medizinisches Pflaster für die Abgabe des antiphlogistisch wirkenden Wirkstoffs Etofenamat an die Haut, wobei dieses Pflaster einen Aufbau aufweist, bestehend aus der Deckschicht (a), der Trägerschicht (c), der abziehbaren Schutzschicht (d), und gegebenenfalls einer Zwischenschicht (b), dadurch gekennzeichnet, dass:
- die Deckschicht (a) aus einem inerten Material besteht;
- die Trägerschicht (c) aus einem selbstklebenden Polysiloxan als Trägermaterial besteht, welches durch Kondensation eines Silanolgruppen enthaltenden Dimethylpolysiloxans mit einem in organischen Lösungsmitteln löslichem Silikatharz und anschliessender Umsetzung der verbleibenden Silanolgruppen mit einer reaktiven Trimethylsilylverbindung erhalten wurde; und

- dieses selbstklebende Polysiloxan gegebenenfalls an sich bekannte Zusatzstoffe für die Modifizierung der Klebeeigenschaften, und gegebenenfalls Zusatzstoffe zur Herabsetzung der Viskosität des selbstklebenden Polysiloxans enthält, und in welche der antiphlogistisch wirkende Wirkstoff, gegebenenfalls zusammen mit einem die Permeation durch die Haut fördernden Mittel in Form einer Dispersion eingelagert ist;
- die Klebekraft der Trägerschicht (c) im Bereich von 0.8 N/25mm bis 1.4 N/25mm liegt;
- die Trägerschicht (c) den Wirkstoff Etofenamat in einer Konzentration im Bereich von 1.0 Gew.-% bis 25.0 Gew.-%, berechnet auf das Gesamtgewicht der Trägerschicht (c), enthält;
- die Trägerschicht (c) den in disperser Form vorliegenden Wirkstoff in einer durchschnittlichen Tröpfchengrösse im Bereich von 0.1 µm bis 500 µm enthält;
- die Trägerschicht (c) direkt an der Deckschicht (a) haftet, oder gegebenenfalls mit dieser über die Zwischenschicht (b) verbunden ist; und
- die abziehbare Schutzschicht (d) aus einem inerten Material besteht, an der Trägerschicht (c) haftet und von dieser leicht abziehbar ist.

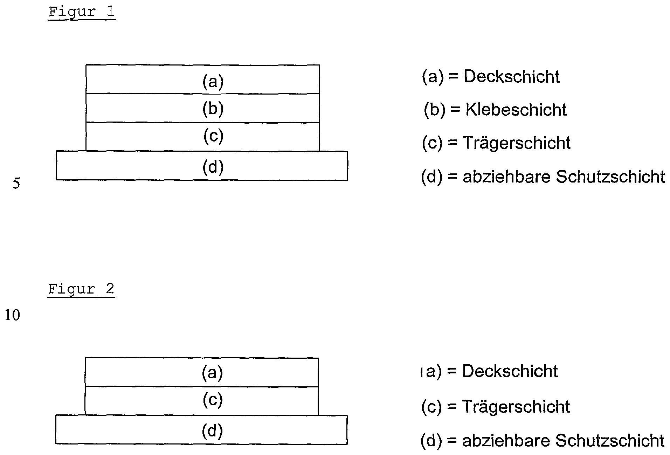

Bevorzugt ist ein medizinisches Pflaster, welches einen Aufbau gemäss Figur 2 aufweist, bestehend aus der Deckschicht (a), der Trägerschicht (c), und der abziehbaren Schutzschicht (d), dadurch gekennzeichnet, dass:
- die Deckschicht (a) aus einem inerten Material besteht,
- die Trägerschicht (c) aus einem selbstklebenden Polysiloxan besteht, in welchem der antiphlogistisch wirkende Wirkstoff Etofenamat, gegebenenfalls zusammen mit einem die Permeation durch die Haut fördernden Mittel und gegebenenfalls weiteren Zusatzstoffen, in Form einer Dispersion eingelagert ist, und diese Trägerschicht (c) direkt an der Deckschicht (a) haftet, und
- die abziehbare Schutzschicht (d) aus einem inerten Material besteht, an der Trägerschicht (c) haftet und von dieser leicht abziehbar ist.

Ist eine Klebeschicht (b) als Zwischenschicht zwischen der Deckschicht (a) und der Trägerschicht (c) vorhanden, so enthält diese Klebeschicht (b) entweder keinen Wirkstoff oder ist gegebenenfalls mit Wirkstoff in unterschiedlichen Mengen beladen und weist eine vergleichsweise starke Klebekraft auf, so dass die Klebeschicht (b) sowohl an der Deckschicht (a) als auch an der Trägerschicht (c) fest haftet. Dabei ist die Klebekraft der Klebeschicht (b) weitgehend unabhängig von der, den Wirkstoff enthaltenden, Trägerschicht (c) und höher als die Klebekraft der Trägerschicht (c). Die Klebekraft der Trägerschicht (c) ist nur so gross, dass das Pflaster leicht und gänzlich von der Haut entfernt werden kann.

Ist die Klebeschicht (b) mit Wirkstoff beladen, so ist der Wirkstoff vorzugsweise in mindestens der gleichen Menge in der Klebeschicht (b) vorhanden, wie der Wirkstoff in der Trägerschicht (c) vorhanden ist. Vorzugsweise ist die Klebeschicht (b) mit dem Wirkstoff bis zur Sättigungsgrenze beladen.

Die vorliegende Erfindung betrifft auch ein Verfahren zur Herstellung des Pflasters gemäss Figur 1 enthaltend eine Klebeschicht (b), dadurch gekennzeichnet, dass man die Bestandteile der Klebeschicht (b), in verflüssigtem Zustand, d.i. als "hot-melt" ohne Zusatz eines Lösungsmittels, oder gelöst in einem geeigneten organischen Lösungsmittel, auf die Deckschicht (a) aufträgt und anschliessend vom gegebenenfalls anwesenden organischen Lösungsmittel befreit bzw. trocknet; anschliessend die Bestandteile der Trägerschicht (c) in verflüssigtem Zustand, d.i. als "hot-melt" ohne Zusatz eines Lösungsmittels, oder gelöst in einem geeigneten organischen Lösungsmittel, auf die Klebeschicht (b) aufträgt und anschliessend vom gegebenenfalls anwesenden organischen Lösungsmittel befreit bzw. trocknet; und auf die getrocknete Trägerschicht (c) die abziehbare Schutzschicht (d) aufbringt. Dabei kann jeweils auch die Klebeschicht (b), wie vorgehend beschrieben, gegebenenfalls den Wirkstoff enthalten.

Die vorliegende Erfindung betrifft auch ein Verfahren zur Herstellung des Pflasters gemäss Figur 2, dadurch gekennzeichnet, dass man die Bestandteile der Trägerschicht (c) in verflüssigtem Zustand, d.i. als "hot-melt" ohne Zusatz eines Lösungsmittels, oder gelöst in einem geeigneten organischen Lösungsmittel, auf die Deckschicht (a) aufträgt und anschliessend vom gegebenenfalls anwesenden organischen Lösungsmittel befreit bzw. trocknet; und auf die getrocknete Schicht (c) die abziehbare Schutzschicht (d) aufbringt.

Man kann bei der Herstellung des erfindungsgemässen Pflasters auch von der abziehbaren Schutzschicht (d) ausgehen. Dann ist das Verfahren zur Herstellung des Pflasters, beispielsweise gemäss Figur 1, dadurch gekennzeichnet, dass man die Bestandteile der Trägerschicht (c), in verflüssigtem Zustand, d.i. als "hot-melt" ohne Zusatz eines Lösungsmittels, oder gelöst in einem geeigneten organischen Lösungsmittel, auf die abziehbare Schutzschicht (d) aufträgt und anschliessend vom gegebenenfalls anwesenden organischen Lösungsmittel befreit bzw. trocknet; in einem separaten Schritt die Bestandteile der Klebeschicht (b), in verflüssigtem Zustand, d.i. als "hot-melt" ohne Zusatz eines Lösungsmittels, oder gelöst in einem geeigneten organischen Lösungsmittel, auf die Deckschicht (a) aufträgt und anschliessend vom gegebenenfalls anwesenden organischen Lösungsmittel befreit bzw. trocknet; und anschliessend die Trägerschicht (c) mit der Deckschicht (a), welche bereits die Klebeschicht (b) enthält, kaschiert.

Analogerweise ist das Verfahren zur Herstellung des Pflasters, beispielsweise gemäss Figur 2, dadurch gekennzeichnet, dass man die Bestandteile der Trägerschicht (c), in verflüssigtem Zustand, d.i. als "hot-melt" ohne Zusatz eines Lösungsmittels, oder gelöst in einem geeigneten organischen Lösungsmittel, auf die abziehbare Schutzschicht (d) aufträgt und anschliessend vom gegebenenfalls anwesenden organischen Lösungsmittel befreit bzw. trocknet; und auf die getrocknete Trägerschicht (c) die Deckschicht (a) aufbringt.

Vorzugsweise trocknet man die Klebeschicht (b) und die Trägerschicht (c) direkt nach dem Aufbringen, insofern diese Lösungsmittel enthalten. Es ist aber auch möglich, alle Schichten nacheinander aufzubringen und diese erst am Ende zu trocknen.

Vorzugsweise verarbeitet man die Trägerschicht als "hot-melt". Hierzu mischt man die Bestandteile der Trägerschicht (c) in verflüssigtem Zustand intensiv, indem man den Wirkstoff ohne Zusatz eines Lösungsmittels, oder gelöst in einem geeigneten organischen Lösungsmittel, gegebenenfalls zusammen mit einem die Permeation durch die Haut fördernden Mittel und gegebenenfalls weiteren Zusatzstoffen, einsetzt. Dabei mischt man die Bestandteile vorzugsweise bei einer Temperatur im Bereich von 80°C-190°C, vorzugsweise im Bereich von 140°C-180°C und insbesondere im Bereich von 160°C-180°C, so lange bis sich die gewünschte Dispersion des Wirkstoffs in der Matrix gebildet hat. Anschliessend wird auf die Laminierungstemperatur, d.h. auf eine Temperatur im Bereich von 120°C-140°C, vorzugsweise im Bereich von 80°C-120°C und insbesondere auf etwa 100°C, abkühlen gelassen, wobei die Dispersion bei dieser Temperatur in Form einer "hot-melt" auf die gewünschte Unterlage aufgetragen, bzw. laminiert, und zur Trägerschicht (c) verarbeitet wird. Bei der Laminierungstemperatur ist die Dispersion vorzugsweise lösungsmittelfrei oder wird anschliessend, vor oder nach der Laminierung, vom gegebenenfalls noch anwesenden organischen Lösungsmittel befreit. Polysiloxane, welche lösungsmittelfrei aufgebracht werden können und Polysiloxane, welche vorzugsweise aus einem Lösungsmittel aufgebracht werden unter anschliessendem Entfernen des Lösungsmittels, sind an sich bekannt.

Die Deckschicht (a) besteht vorzugsweise aus einem elastischen textilen Flächengebilde, welches mit einem polymeren Material beschichtet ist. Als textiles Material verwendet man z.B. ein textiles Flächengebilde, welches beispielsweise aus Baumwolle gefertigt und mit Polyethylenterephthalat (PET) beschichtet ist. Das textile Flächengebilde kann auch aus einer synthetischen textilen Faser, wie beispielsweise PET, PVC, PUR und weiteren polymeren Kunststoffen, bestehen oder in nicht-gewobener Form gefertigt sein. Solche Materialien sind an sich bekannt und kommerziell erhältlich.

Die Klebeschicht (b) besteht aus einem, vorzugsweise in einem organischen Lösungsmittel löslichen, an sich bekannten organischen Polymeren mit guten Klebeeigenschaften. Geeignete Klebematerialien sind beispielsweise Polymerisate von Isopren oder Copolymerisate von Isopren mit Styrol wie Styrol-Isopren-Styrol (SIS); Polyalkylacrylate, hergestellt aus beispielsweise Amyl-, Butyl-, Hexyl-, Heptyl-, Octyl-, Nonyl-, 2-Ethylhexyl-, oder 2-Methoxyethyl-acrylat oder Copolymere von solchen Alkylacrylaten mit Acrylsäure, Methacrylsäure, Methyl- oder Ethylacrylat, Hydroxyethylacrylat oder Hydroxypropylacrylat. Geeignet sind auch Styrol-Butadiene-Styrol-Copolymere (SBS) oder Polyisobutylene und Copolymere davon.

Besonders bevorzugt für die Herstellung der Klebeschicht (b) sind Copolymere hergestellt aus 2-Ethylhexylacrylat und Methylacrylat, wie beispielsweise aus etwa 19.9 Gew.-% 2-Ethylhexylacrylat und etwa 79.3 Gew.-% Methylacrylat, mit einem durchschnittlichen Molekulargewicht im Bereich von 350'000 bis 550'000 Dalton, vorzugsweise etwa 400'000 bis 500'000 Dalton, und polymerisiert z.B. in Gegenwart von Azobisisobutyronitril. Die genannten Acrylatpolymere werden vorzugsweise auch in Kombination mit SBS-Polymeren verwendet, wobei das Verhältnis vom Fachmann optimiert werden kann. So kann beispielsweise das Gewichtsverhältnis des Acrylatpolymeren zu SBS im Bereich von 2:1 bis 1:2, vorzugsweise im Bereich von 5:3 bis 3:5, liegen.

Bevorzugt für die Herstellung der Klebeschicht (b) sind auch Styrol-Butadien-Styrol Blockcopolymere (SBS), Styrol-Butadien-Blockcoplymere (SB) und Mischungen dieser Copolymeren mit einer Glasübergangstemperatur (T_{G}) von vorzugsweise weniger als -22°C [T_{G}<(-22°C)]. Diese Polymere können Zusatzstoffe, wie z.B. den Glycerinester von hydriertem Kolophonium oder Polyterpene enthalten. Eine bevorzugte Zusammensetzung enthält beispielsweise 17.0 Gew.-% SB, 11.3 Gew.-% SBS, 70.8 Gew.-% des Kolophonium-Glycerinesters und 0.9 Gew.-% eines Antioxydans. Bevorzugte Lösungsmittel für diese Klebematerialien sind beispielsweise gesättigte und aromatische Kohlenwasserstoffe, wie z.B. Hexan, Heptan, Oktan, Benzol oder Toluol.

Für die Herstellung der Klebeschicht (b) werden die Polymere und/oder Copolymere vorzugsweise in einem geeigneten Lösungsmittel gelöst. Enthält die Klebeschicht (b) den Wirkstoff, so wird der Wirkstoff vorzugsweise vorgehend in der gewünschten Menge in einem geeigneten Lösungsmittel gelöst und in gelöster Form mit dem Polymeren und/oder Copolymeren der Klebeschicht (b) vermischt. Die Lösung der Klebeschicht (b) wird auf die Deckschicht (a) aufgebracht und das Lösungsmittel entfernt. Geeignete Lösungsmittel sind beispielsweise Ethylacetat, Propylacetat, gesättigte und aromatische Kohlenwasserstoffe, wie z.B. Hexan, Heptan, Oktan, Benzol oder Toluol.

Zahlreiche der für die Herstellung der Klebeschicht (b) geeigneten Polymeren können auch in verflüssigtem Zustand, ohne den Zusatz eines Lösungsmittels, als "hot-melt" aufgebracht werden. Solche, für die Herstellung der Klebeschicht (b) verwendbaren Polymere und Copolymere sind an sich bekannt und kommerziell erhältlich. Beschichtete Flächengebilde sind beispielsweise erhältlich als Scotchpak® der Firma 3M. Beispielsweise wird die Deckschicht in einem ersten Schritt mit etwa 40g/m² SBS (als Klebeschicht) beschichtet. Scotchpak wird beispielsweise als Schutzschicht verwendet (z.B. Scotchpak 1022 der Firma 3M). Ob die Klebeschicht (b) auf die Deckschicht (a) aus einem Lösungsmittel oder lösungsmittelfrei aufgebracht wird ist erfindungsgemäss unwesentlich. Vorzugsweise enthält das erfindungsgemässe Pflaster keine Klebeschicht (b).

Die Trägerschicht (c) besteht erfindungsgemäss aus einem selbstklebenden Polysiloxan. Selbstklebenden Polysiloxane sind an sich bekannt und werden beispielsweise von der Firma Dow Corning unter dem Markennamen BIO-PSA® Amine-Compatible Silicone Adhesives in unterschiedlichen Zusammensetzungen hergestellt und kommerziell vertrieben. Solche Silikonpolymere sind erfindungsgemäss verwendbar. Solche Silikonpolymere können zur Optimierung der Klebeeigenschaften zusätzlich an sich bekannte Zusatzstoffe für die Modifizierung der Klebeeigenschaften, wie z.B. Kolophoniumverbindungen, enthalten, wie z.B. dehydriertes oder hydriertes Kolophonium, Kolophonium-Glycerinester, Terpenharze, Polyterpenharze aus alpha- oder beta-Pinen, oder niedrig-viskose Silikone bzw. Polysiloxane, welche endständige Silanolgruppen aufweisen, oder Polydimethylsiloxane wie z.B. Dimethiconol.

Selbstklebende Polysiloxane, welche für die Herstellung der Trägerschicht (c) ohne Zusatz eines Lösungsmittels als "hot-melt" geeignet sind, sind kommerziell erhältlich, beispielsweise als BIO-PSA® 7-4560 Silicone Adhesive der Firma Dow Corning bekannt. Solche Siloxanverbindungen sind z.B. unter CAS-Nummer 68440-70-0 und CAS-Nummer 63148-62-9 bekannt und deren Gemische, z.B. Verbindungen der CAS-Nummer 68440-70-0 in einer Konzentration von mindestens 60 Gew.-% mit Verbindungen der CAS-Nummer 63148-62-9 in einer Konzentration von 10.0 bis 30.0 Gew.-%.

Selbstklebende Polysiloxane, welche für die Herstellung der Trägerschicht (c) unter Zusatz eines Lösungsmittels geeignet sind, sind kommerziell erhältlich, beispielsweise als BIO-PSA® 7-4603 oder BIO-PSA® 7-4201 der Firma Dow Corning. Geeignete Polysiloxane für die Herstellung der Trägerschicht (c) sind für den Fachmann ohne weiteres auswählbar.

Geeignete selbstklebende Polysiloxane werden beispielsweise durch Kondensation eines Silanolgruppen enthaltenden Dimethylpolysiloxans mit einem in organischen Lösungsmitteln löslichem Silikatharz und anschliessender Umsetzung der verbleibenden Silanolgruppen mit einer reaktiven Trimethylsilylverbindung erhalten. Solche Polysiloxane sind löslich in organischen Lösungsmitteln, wie beispielsweise in Ethylacetat, Propylacetat, gesättigten und aromatischen Kohlenwasserstoffen, wie z.B. Hexan, Heptan, Oktan, Benzol oder Toluol.

Eine bevorzugte Ausführungsform besteht darin, dass das selbstklebende Polysiloxan der Trägerschicht (c) eine Verbindung oder ein Gemisch von Verbindungen enthält, welche die Viskosität des den Wirkstoff enthaltenden selbstklebenden Polysiloxans herabsetzen, ohne die übrigen Eigenschaften des selbstklebenden Polysiloxans negativ zu beeinflussen. Solche Verbindung sind vorzugsweise Glycerin und/oder Esterverbindungen einer mittelkettigen Fettsäure mit einem einwertigen oder mehrwertigen Alkohol. Bevorzugte Esterverbindungen einer mittelkettigen Fettsäure mit einem einwertigen Alkohol sind beispielsweise Ester von Propylalkohol, Isopropylalkohol, Butylalkohol oder Isopropylalkohol mit einer (C₁₀-C₁₆)-Fettsäure, vorzugsweise mit mittelkettigen (C₁₂-C₁₆)-Fettsäuren, vorzugsweise mit Laurinsäure, Myristinsäure oder Palmitinsäure, wie beispielsweise Isopropylmyristat. Esterverbindungen von mittelkettigen Fettsäuren mit mehrwertigen Alkoholen sind beispielweise Mono-, Di-, oder Triester von Glycerin mit mittelkettigen (C₁₀-C₁₆) -Fettsäuren, vorzugsweise Mono-, Di-, oder Triester von Glycerin mit mittelkettigen (C₁₂-C₁₆)-Fettsäuren sowie auch natürliche Öle, vorzugsweise Olivenöl oder Rizinusöl. Als Verbindungen, welche die Viskosität des den Wirkstoff enthaltenden selbstklebenden Polysiloxans analogerweise herabsetzen, können auch flüssiges Paraffin, Polysorbate (d.h. Polyoxyethylen-Sorbitan-Fettsäureesterverbindungen), wie zum Beispiel Tween®60 (Polyoxyethylen-Sorbitan-Monostearat) oder Tween®80 (Polyoxyethylen-Sorbitan-Monooleat), Polyethylenglykole, wie z.B. Polyethylenglykol 400, Propylenglykol und Polypropylenglykole, Ester von mehrwertigen Säuren mit Alkoholen wie Triethylcitrat, und Mischungen dieser Verbindungen verwendet werden. Die erfindungsgemäss verwendeten selbstklebenden Polysiloxane enthalten vorzugsweise etwa 2-15 Gew.-%, vorzugsweise etwa 5-10 Gew.-% dieser Verbindungen, bezogen auf das Gesamtgewicht der Trägerschicht (c). Die genannten Verbindungen können fallweise auch als Permeations-Beschleuniger wirken.
- Die den Wirkstoff enthaltende Trägerschicht (c) sowie auch die gegebenenfalls den Wirkstoff enthaltende Klebeschicht (b), enthalten den Wirkstoff gegebenenfalls zusammen mit einem die Permeation durch die Haut fördernden Mittel. Die Anwesenheit eines die Permeation durch die Haut fördernden Mittels ist jedoch nicht kritisch. Der Wirkstoff kann zusätzlich auch mit weiteren Wirkstoffen gemischt sein und zusätzlich z.B. Stabilisatoren und Riechstoffe enthalten.

Bevorzugt ist Etofenamat, entsprechend der chemischen Formel: 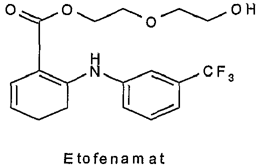 d.i. 2-(2-Hydroxyethoxy)-ethyl-(α,α,α-trifluor-m-tolyl)-anthranilat (Etofenamat).

Die Trägerschicht (c) enthält den Wirkstoff, vorzugsweise Etofenamat, vorzugsweise in einer Konzentration im Bereich von etwa 1.0 Gew.-% bis etwa 25.0 Gew.-%, vorzugsweise 2.0 Gew.-% bis 20 Gew.-%, und vorzugsweise 2.5 Gew.-% bis 15 Gew.-%, vorzugsweise in einer Konzentration von etwa 5 Gew.-% bis etwa 10 Gew.-%, berechnet auf das Gesamtgewicht der Trägerschicht.

Ein Verfahren zur Herstellung der Dispersion verwendbar als Trägerschicht (c), enthaltend mindestens ein selbstklebendes Polysiloxan sowie den Wirkstoff, vorzugsweise Etofenamat, gegebenenfalls zusammen mit einem die Permeation durch die Haut fördernden Mittel und gegebenenfalls weiteren Zusatzstoffen, ist dadurch gekennzeichnet, dass man das die Trägerschicht (c) bildende selbstklebende Polysiloxan zusammen mit dem Wirkstoff, gegebenenfalls zusammen mit einem die Permeation durch die Haut fördernden Mittel und gegebenenfalls weiteren Zusatzstoffen, auf eine Temperatur im Bereich von 80°C-200°C, vorzugsweise im Bereich von 140°C-190°C und insbesondere im Bereich von 160°C-190°C, unter intensiven Rühren so lange erhitzt, bis sich die gewünschte Dispersion gebildet hat. Diese Dispersion lässt sich im angegebenen Temperaturbereich, jedoch vorzugsweise im Bereich von 120°C-140°C, vorzugsweise im Bereich von 80°C-120°C und insbesondere bei etwa 100°C, lösungsmittelfrei weiter verarbeiten und als dünne Schicht in der gewünschten Menge auf die vorgesehene Unterlage auftragen.

Ein weiteres Verfahren zur Herstellung der Dispersion verwendbar als Trägerschicht (c), enthaltend mindestens ein selbstklebendes Polysiloxan sowie den Wirkstoff, vorzugsweise Etofenamat, gegebenenfalls zusammen mit einem die Permeation durch die Haut fördernden Mittel und gegebenenfalls weiteren Zusatzstoffen, ist dadurch gekennzeichnet, dass man das die Trägerschicht (c) bildende selbstklebende Polysiloxan zusammen mit dem Wirkstoff, gegebenenfalls zusammen mit einem die Permeation durch die Haut fördernden Mittel und gegebenenfalls weiteren Zusatzstoffen, in Gegenwart eines Lösungsmittels bei erhöhter Temperatur, vorzugsweise im Bereich von 40°C-90°C, vorzugsweise im Bereich des Siedepunkts des Lösungsmittels, unter intensivem Rühren so lange erhitzt, bis sich die gewünschte Dispersion gebildet hat. Vorzugsweise setzt man so viel Lösungsmittel zu, dass sich die erhaltene Dispersion bei Raumtemperatur zu einem Pflaster verarbeiten lässt, d.h. sich die erhaltene Dispersion bei Raumtemperatur als dünne Schicht in der gewünschten Menge auf die vorgesehene Unterlage auftragen lässt.

Alternativ ist es möglich, das Lösungsmittel aus der gebildeten Dispersion [zur Bildung der Trägerschicht (c)] weitgehend oder gänzlich zu entfernen und die derart gebildete Dispersion bei erhöhter Temperatur weitgehend oder gänzlich lösungsmittelfrei weiter zu verarbeiten und als dünne Schicht in der gewünschten Menge auf die vorgesehene Unterlage aufzutragen.

Als Lösungsmittel verwendet man vorzugsweise ein organisches Lösungsmittel, wie beispielsweise Ethylacetat, Propylacetat, gesättigte und/oder aromatische Kohlenwasserstoffe, wie z.B. Hexan, Heptan, Oktan, Benzol oder Toluol. Das Lösungsmittel wird vorzugsweise bei einer Temperatur im Bereich von 50°C bis 90°C, je nach Siedepunkt des Lösungsmittels, vorzugsweise bei einer Temperatur von etwa 60°C bis 70°C, während einer Zeit von etwa 30 Minuten bis 60 Minuten, entweder aus der erhaltenen Dispersion oder aus der im Pflaster aufgetragenen Schicht, abgedampft.

Die Trägerschicht (c) enthält den Wirkstoff in hochdisperser Verteilung mit einer durchschnittlichen Tröpfchengrösse im Bereich von 0.1 µm bis 500 µm, vorzugsweise von 1.0 µm bis 100 µm. Die Belegung der Fläche für die Klebeschicht (b) und die Trägerschicht (c) liegt jeweils im Bereich von 30 g/m² bis 300 g/m², vorzugsweise im Bereich von etwa 40 g/m² bis 200 g/m², und insbesondere im Bereich von etwa 40 g/m² bis 100 g/m².

Die Klebekraft der Klebeschicht (b) liegt vorzugsweise im Bereich von 0.8 N/25mm bis 2.0 N/25mm, vorzugsweise im Bereich von 0.9 N/25mm bis 1.7 N/25mm. Die Klebekraft der Trägerschicht (c) liegt vorzugsweise im Bereich von 0.8 N/25mm bis 1.4 N/25mm.

Verbindungen, welche die Permeation durch die Haut fördern (Permeations-Enhancer), sind Zusatzstoffe, welche die Verabreichung des Wirkstoffs an die Haut, bzw. die Durchdringung des Stratum Corneums, fördern. Solche Verbindungen sind an sich, sowie auch für die Verwendung in solchen Pflastern, bekannt. Bevorzugt sind natürlich vorkommende Stoffe, wie natürliche Öle und Fette oder Fettsäuren und höhere Fettalkohole und Ester derselben sowie Glycerin und Gemische dieser Verbindungen. Das Gewichtsverhältnis von Wirkstoff : Permeations-Enhancer liegt vorzugsweise im Bereich von 98 : 2 bis 2:8, vorzugsweise im Bereich von 9 : 1 bis 3 : 7, vorzugsweise bei etwa 1 : 2.

Natürliche Öle und Fette sind insbesondere Mono-, Di- und Triglyceride, welche Glyceridester mit gesättigten und/oder ungesättigten Fettsäuren darstellen; beispielsweise Ester von Fettsäuren mit vorzugsweise 4 bis 22 Kohlenstoffatomen. Solche Fettsäuren sind vorzugsweise Butter-, Capron-, Caprin-, Myristin-, Palmitin-, Stearin-, Arachin-, Palmitolein-, Olein(Öl-)-, Ricinolein-, Linol-, Linolen-, Arachidonsäure. Solche Fettsäuren mit vorzugsweise 4 bis 22 Kohlenstoffatomen können auch alleine als Beschleuniger eingesetzt werden.

Unter Alkoholen sind die entsprechenden Alkohole und Fettalkohole mit 4 bis 22 Kohlenstoffatomen zu verstehen, vorzugsweise n-, iso-, sec.-Butylalkohol; n-, iso-, tert.-Amylalkohol; n-Hexylalkohol, cyclo-Hexylalkohol; Octylalkohol, Caprylalkohol (Octanol-2), n-Decylalkohol, Laurylalkohol, Myristylalkohol, Cetylakohol, Stearylalkohol.

Geeignet sind auch synthetische Fettsäureester der genannten Fettsäuren mit niedrigen oder höheren Alkoholen, wie beispielsweise Ethylstearat, Palmitinsäurecetylester, Isopropylmyristat, Isopropylpalmitat oder Gemische solcher Verbindungen. Bevorzugt ist Isopropylmyristat.

Natürliche Öle sind auch z.B. Rizinusöl, Olivenöl, Erdnussöl, Maisöl, Haselnussöl, Jojobaöl, und Weizenkeimöl.

Die abziehbare Schutzschicht (d) besteht aus einem inerten Material, welches an der Trägerschicht (c) haftet und von dieser leicht abziehbar ist. Solche Materialien sind in Form dünner Folien bekannt, und kommerziell beispielsweise von der Firma 3M unter der Marke Scotchpak® erhältlich. Die folgenden Beispiele illustrieren die Erfindung ohne diese zu beschränken.

### Beispiel 1

Zu 5.0 Teilen reinem Etofenamat werden 5.0 Teile reines Ethylacetat in einem Glasrundkolben zugegeben und auf einer Magnetrührplatte intensiv gemischt. Dann werden 85 Teile eines selbstklebenden Polysiloxans gelöst in Ethylacetat (BIO-PSA® 7-4603, respektive BIO-PSA® 7-4560 der Firma Dow Corning) zugegeben und intensiv während 2.5 Stunden bei Raumtemperatur gerührt. Gegebenfalls werden der Mischung 10 Teile Isopropylmyristat (IPM) hinzugefügt. Aus der erhaltenen Mischung wird mit einer Beschichtungsanlage ein Laminat mit einem Flächengewicht von 100 g/m², 75 g/m² und 40g/m² hergestellt [= Trägerschicht (c) auf abziehbarer Schicht (d)], welches im Trockenschrank bei einer Temperatur von 60°C während 60 Minuten getrocknet wird, bis sämtliche Lösungsmittel entfernt sind. Nach der Trocknung wird das erhaltene Laminat mit einer Deckschicht (a) bestehend aus PET-Gewebe, welche mit einer Klebeschicht (b) (Duro-Tak® 87-6173 der Firma National Starch) versehen ist, kaschiert, wobei die Klebeschicht ein Flächengewicht von 40 g/m² aufweist. In analoger Weise wurden die zusätzlich Beispiele der Tabelle 1 hergestellt.

**Tabelle 1**

| Etofenamat | Bio PSA 7-4603 | IPM | area mass |
|---|---|---|---|
| 5% | 95% | - | 40g/m² |
| 5% | 95% | - | 75g/m² |
| 10% | 90% | - | 40g/m² |
| 10% | 90% | - | 75g/m² |
| 5% | 85% | 10% | 100g/m² |
| 10% | 80% | 10% | 100g/m² |

### Beispiel 2

5.0 Teile reines Etofenamat werden mit 10 Teilen Isopropylmyristat (IPM) und 85 Teilen eines selbstklebenden Polysiloxans (BIO-PSA® 4560 der Firma Dow Corning) in einem Hochgeschwindigkeitsmischer der Marke Becomix bei einer Temperatur von 190°C intensiv während 2.5 Stunden gerührt. Die erhaltene Mischung bzw. Dispersion wird abkühlen gelassen und mit einer Beschichtungsanlage der Firma Hofmann & Schwabe bei einer Laminierungstemperatur von 100°C zu einem Laminat mit einem Flächengewicht von 100 g/m², 75 g/m² und von 40g/m² verarbeitet [= Trägerschicht (c) auf der Deckschicht (a), bestehend aus PET-Gewebe], welches auf Raumtemperatur abgekühlt wird. Dann wird das erhaltene Laminat mit einer Schutzschicht (d) (Scotchpack®, ein mit einem Floropolymer beschichtete entfernbare Folie der Firma 3M) kaschiert.

## Patentansprüche

1. Medizinisches Pflaster für die Abgabe des bei Raumtemperatur flüssigen antiphlogistisch wirkenden Wirkstoffs Etofenamat an die Haut, wobei dieses Pflaster einen Aufbau aufweist, bestehend aus der Deckschicht (a), der Trägerschicht (c), der abziehbaren Schutzschicht (d), und gegebenenfalls einer Zwischenschicht (b), **dadurch gekennzeichnet, dass**:
- die Deckschicht (a) aus einem inerten Material besteht;
- die Trägerschicht (c) aus einem selbstklebenden Polysiloxan als Trägermaterial besteht, welches durch Kondensation eines Silanolgruppen enthaltenden Dimethylpolysiloxans mit einem in organischen Lösungsmitteln löslichem Silikatharz und anschliessender Umsetzung der verbleibenden Silanolgruppen mit einer reaktiven Trimethylsilylverbindung erhalten wurde; und
- dieses selbstklebende Polysiloxan gegebenenfalls an sich bekannte Zusatzstoffe für die Modifizierung der Klebeeigenschaften, und gegebenenfalls Zusatzstoffe zur Herabsetzung der Viskosität des selbstklebenden Polysiloxans enthält, und in welche der antiphlogistisch wirkende Wirkstoff, gegebenenfalls zusammen mit einem die Permeation durch die Haut fördernden Mittel in Form einer Dispersion eingelagert ist;
- die Klebekraft der Trägerschicht (c) im Bereich von 0.8 N/25mm bis 1.4 N/25mm liegt;
- die Trägerschicht (c) den Wirkstoff Etofenamat in einer Konzentration im Bereich von 1.0 Gew.-% bis 25.0 Gew.-%, berechnet auf das Gesamtgewicht der Trägerschicht (c), enthält;
- die Trägerschicht (c) den in disperser Form vorliegenden Wirkstoff in einer durchschnittlichen Tröpfchengrösse im Bereich von 0.1 µm bis 500 µm enthält;
- die Trägerschicht (c) direkt an der Deckschicht (a) haftet, oder gegebenenfalls mit dieser über die Zwischenschicht (b) verbunden ist; und
- die abziehbare Schutzschicht (d) aus einem inerten Material besteht, an der Trägerschicht (c) haftet und von dieser leicht abziehbar ist.

2. Pflaster nach Anspruch 1, welches einen Aufbau aufweist, bestehend aus der Deckschicht (a), der Trägerschicht (c), und der abziehbaren Schutzschicht (d), **dadurch gekennzeichnet, dass**:
- die Deckschicht (a) aus einem inerten Material besteht,
- die Trägerschicht (c) aus einem selbstklebenden Polysiloxan besteht, in welchem der antiphlogistisch wirkende Wirkstoff, gegebenenfalls zusammen mit einem die Permeation durch die Haut fördernden Mittel und gegebenenfalls weiteren Zusatzstoffen, in Form einer Dispersion eingelagert ist, und diese Trägerschicht (c) direkt an der Deckschicht (a) haftet, und
- die abziehbare Schutzschicht (d) aus einem inerten Material besteht, an der Trägerschicht (c) haftet und von dieser leicht abziehbar ist.

3. Pflaster nach Anspruch 1, **dadurch gekennzeichnet, dass** eine Zwischenschicht (b) zwischen der Deckschicht (a) und der Trägerschicht (c) vorhanden ist, diese Klebeschicht (b) entweder keinen Wirkstoff enthält oder mit Wirkstoff in unterschiedlicher Menge beladen ist, und die Klebekraft der Klebeschicht (b) höher ist als die Klebekraft der Trägerschicht (c), wobei die Klebekraft der Trägerschicht (c) nur so gross ist, dass das Pflaster leicht und gänzlich von der Haut entfernt werden kann.

4. Pflaster nach Anspruch 3, **dadurch gekennzeichnet, dass** die Klebeschicht (b) mit Wirkstoff beladen ist und der Wirkstoff in mindestens der gleichen Menge in der Klebeschicht (b) vorhanden ist, wie der Wirkstoff in der Trägerschicht (c) vorhanden ist, wobei vorzugsweise die Klebeschicht (b) mit dem Wirkstoff bis zur Sättigungsgrenze beladen ist.

5. Pflaster nach einem der Ansprüche 1-4, **dadurch gekennzeichnet, dass** die Deckschicht (a) aus einem elastischen textilen Flächengebilde, welches mit einem polymeren Material beschichtet ist, besteht.

6. Pflaster nach einem der Anspruch 1, oder 3-5, **dadurch gekennzeichnet, dass** die Klebeschicht (b) aus einem an sich bekannten, vorzugsweise in einem organischen Lösungsmittel löslichen, organischen Polymeren mit guten Klebeeigenschaften, besteht, vorzugsweise ausgewählt aus der Gruppe enthaltend: 2-Ethylhexylacrylat, Methylacrylat, SBS-Polymere, Styrol-Butadien-Styrol Blockcopolymere (SBS), Styrol-Butadien-Blockcoplymere (SB) und Mischungen dieser Copolymeren mit einer Glasübergangstemperatur (T_{G}) von vorzugsweise weniger als -22°C [T_{G}<(-22°C)], und diese Polymere gegebenenfalls Zusatzstoffe enthalten, vorzugsweise den Glycerinester von hydriertem Kolophonium oder Polyterpene.

7. Pflaster nach Anspruch 6, **dadurch gekennzeichnet, dass** die Klebeschicht (b) vorwiegend aus 2-Ethylhexylacrylat und Methylacrylat besteht, vorzugsweise aus etwa 19.9 Gew.-% 2-Ethylhexylacrylat und etwa 79.3 Gew.-% Methylacrylat, mit einem durchschnittlichen Molekulargewicht im Bereich von 350'000 bis 550'000 Dalton, vorzugsweise etwa 400'000 bis 500'000 Dalton.

8. Pflaster nach Anspruch 6, **dadurch gekennzeichnet, dass** die Klebeschicht (b) aus 17.0 Gew.-% SB, 11.3 Gew.-% SBS, 70.8 Gew.-% des Kolophonium-Glycerinesters und 0.9 Gew.-% eines Antioxydans, besteht.

9. Pflaster nach einem der Ansprüche 1-8, **dadurch gekennzeichnet, dass** die Trägerschicht (c) aus einem selbstklebenden Polysiloxan besteht, welches ausgewählt ist aus selbstklebenden Polysiloxanen registriert als Dow Corning® BIO-PSA® 7-4201, Dow Corning® BIO-PSA 7-4560 und Dow Corning® BIO-PSA 7-4603.

10. Pflaster nach einem der Ansprüche 1-9, **dadurch gekennzeichnet, dass** die Zusatzstoffe für die Modifizierung der Klebeeigenschaften ausgewählt sind aus Kolophoniumverbindungen, vorzugsweise dehydriertes oder hydriertes Kolophonium, Kolophonium-Glycerinester, Terpenharze, Polyterpenharze, aus alpha- oder beta-Pinen, oder ein Gemisch dieser Verbindungen, oder aus niedrig-viskosen Silikonen bzw. Polysiloxanen, welche endständige Silanolgruppen aufweisen.

11. Pflaster nach Anspruch 10, **dadurch gekennzeichnet, dass** die Trägerschicht (c) aus einem selbstklebenden Polysiloxan besteht, welches bei der Herstellung des Pflasters ohne Zusatz eines Lösungsmittels verwendet, bzw. auf die Unterlage aufgebracht, werden kann.

12. Pflaster nach einem der Ansprüche 1-11, **dadurch gekennzeichnet, dass** das selbstklebende Polysiloxan der Trägerschicht (c) mindestens eine Verbindung zur Herabsetzung der Viskosität des selbstklebenden Polysiloxans enthält, welche ausgewählt ist aus der Gruppe enthaltend Glycerin und/oder eine Esterverbindung einer mittelkettigen Fettsäure mit einem einwertigen Alkohol, vorzugsweise einen Ester von Propylalkohol, Isopropylalkohol, Butylalkohol oder Isopropylalkohol mit einer (C₁₀-C₁₆)-Fettsäure, vorzugsweise mit einer mittelkettigen (C₁₂-C₁₆)-Fettsäure, vorzugsweise mit Laurinsäure, Myristinsäure oder Palmitinsäure, vorzugsweise Isopropylmyristat, wobei vorzugsweise etwa 2-15 Gew.-%, vorzugsweise etwa 5-10 Gew.-% dieser Verbindungen, bezogen auf das Gesamtgewicht der Trägerschicht (c) anwesend ist.

13. Pflaster nach einem der Ansprüche 1-11, **dadurch gekennzeichnet, dass** das selbstklebende Polysiloxan der Trägerschicht (c) mindestens eine Verbindung zur Herabsetzung der Viskosität des selbstklebenden Polysiloxans enthält, welche ausgewählt ist aus der Gruppe enthaltend Esterverbindungen einer mittelkettigen Fettsäuren mit einem mehrwertigen Alkohol enthält, vorzugsweise Mono-, Di-, oder Triester von Glycerin mit mittelkettigen (C₁₀-C₁₆)-Fettsäuren, vorzugsweise Mono-, Di-, oder Triester von Glycerin mit mittelkettigen (C₁₂-C₁₆)-Fettsäuren und/oder natürliche Öle, vorzugsweise Olivenöl oder Rizinusöl, wobei vorzugsweise etwa 2-15 Gew.-%, vorzugsweise etwa 5-10 Gew.-% dieser Verbindungen, bezogen auf das Gesamtgewicht der Trägerschicht (c) anwesend ist.

14. Pflaster nach einem der Ansprüche 1-11, **dadurch gekennzeichnet, dass** das selbstklebende Polysiloxan der Trägerschicht (c) mindestens eine Verbindung zur Herabsetzung der Viskosität des selbstklebenden Polysiloxans enthält, welche ausgewählt ist aus der Gruppe enthaltend flüssiges Paraffin, Polyoxyethylen-Sorbitan-Fettsäureesterverbindungen, vorzugsweise Polyoxyethylen-Sorbitan-Monostearat, Polyoxyethylen-Sorbitan-Monooleat, Polyethylenglykol, vorzugsweise Polyethylenglykol 400, Propylenglykol, Polypropylenglykol, Ester von mehrwertigen Säuren mit Alkoholen, vorzugsweise Triethylcitrat, und Mischungen dieser Verbindungen enthält, wobei vorzugsweise etwa 2-15 Gew.-%, vorzugsweise etwa 5-10 Gew.-%, dieser Verbindungen, bezogen auf das Gesamtgewicht der Trägerschicht (c) anwesend ist.

15. Pflaster nach einem der Ansprüche 1-14, **dadurch gekennzeichnet, dass** die Trägerschicht (c) mindestens eine die Permeation durch die Haut fördernde Verbindung und gegebenenfalls zusätzlich Stabilisatoren und Riechstoffe enthält.

16. Pflaster nach Anspruch 15, **dadurch gekennzeichnet, dass** die Verbindung, welche die Permeation durch die Haut fördert (Permeations-Enhancer) ausgewählt ist aus natürlich vorkommenden Stoffen, vorzugsweise aus natürlichen Ölen und Fetten oder aus Fettsäuren und höheren Fettalkoholen und Ester derselben sowie aus Glycerin und Gemischen dieser Verbindungen, wobei das Gewichtsverhältnis von Wirkstoff:Permeations-Enhancer vorzugsweise im Bereich von 98:2 bis 2:8, vorzugsweise im Bereich von 9:1 bis 3:7, vorzugsweise bei etwa 1 : 2, liegt.

17. Pflaster nach einem der Ansprüche 1-16, **dadurch gekennzeichnet, dass** die Trägerschicht (c) den Wirkstoff Etofenamat, in einer Konzentration im Bereich von 2.0 Gew.-% bis 20 Gew.-%, und vorzugsweise 2.5 Gew.-% bis 15 Gew.-%, vorzugsweise in einer Konzentration von etwa 5 Gew.-% bis etwa 10 Gew.-%, berechnet auf das Gesamtgewicht der Trägerschicht (c) enthält.

18. Pflaster nach einem der Ansprüche 1-17, **dadurch gekennzeichnet, dass** die Trägerschicht (c) den Wirkstoff in disperser Form mit einer durchschnittlichen Tröpfchengrösse im Bereich von 1.0 µm bis 100 µm, enthält.

19. Pflaster nach einem der Ansprüche 1-18, **dadurch gekennzeichnet, dass** die Belegung der Fläche für die Trägerschicht (c) im Bereich von 30 g/m² bis 300 g/m², vorzugsweise im Bereich von 40 g/m² bis 200 g/m², vorzugsweise im Bereich von 40 g/m² bis 100 g/m² liegt.

20. Verfahren zur Herstellung der Dispersion verwendbar als Trägerschicht (c) nach einem der Ansprüche 1-19, **dadurch gekennzeichnet, dass** man das die Trägerschicht (c) bildende selbstklebende Polysiloxan zusammen mit dem Wirkstoff, gegebenenfalls zusammen mit einem die Permeation durch die Haut fördernden Mittel und gegebenenfalls weiteren Zusatzstoffen, bei einer Temperatur im Bereich von 80°C-190°C, vorzugsweise im Bereich von 140°C-180°C und insbesondere im Bereich von 160°C-180°C, so lange mischt, bis sich die gewünschte Dispersion des Wirkstoffs in der Matrix gebildet hat, anschliessend auf eine Temperatur im Bereich von 120°C-140°C, vorzugsweise im Bereich von 80°C-120°C und insbesondere auf etwa 100°C, abkühlen lässt, die Dispersion bei dieser Temperatur in Form einer "hot-melt" auf die gewünschte Unterlage aufträgt und zur Trägerschicht (c) verarbeitet, und gegebenenfalls vor oder nach der Laminierung, vom gegebenenfalls noch anwesenden organischen Lösungsmittel befreit.

21. Verfahren zur Herstellung der Dispersion verwendbar als Trägerschicht (c) nach einem der Ansprüche 1-19, **dadurch gekennzeichnet, dass** man das die Trägerschicht (c) bildende selbstklebende Polysiloxan zusammen mit dem Wirkstoff, gegebenenfalls zusammen mit einem die Permeation durch die Haut fördernden Mittel und gegebenenfalls weiteren Zusatzstoffen, in Gegenwart von genügend Lösungsmittel bei erhöhter Temperatur, vorzugsweise im Bereich von 40°C-90°C, vorzugsweise im Bereich des Siedepunkts des Lösungsmittels, unter intensivem Rühren so lange erhitzt, bis sich die gewünschte Dispersion gebildet hat und dass man anschliessend das Lösungsmittel aus der gebildeten Dispersion weitgehend oder gänzlich entfernt.

22. Verfahren zur Herstellung des Pflasters nach Anspruch 1, **dadurch gekennzeichnet, dass** man die Bestandteile der Klebeschicht (b), in verflüssigtem Zustand oder gelöst in einem geeigneten organischen Lösungsmittel, auf die Deckschicht (a) aufträgt und anschliessend vom gegebenenfalls anwesenden organischen Lösungsmittel befreit bzw. trocknet; anschliessend die Dispersion der Trägerschicht (c) hergestellt gemäss einem der Ansprüche 19 oder 20 auf die Klebeschicht (b) aufträgt und anschliessend vom gegebenenfalls anwesenden organischen Lösungsmittel befreit bzw. trocknet; und auf die getrocknete Trägerschicht (c) die abziehbare Schutzschicht (d) aufbringt.

23. Verfahren zur Herstellung des Pflasters nach Anspruch 2, **dadurch gekennzeichnet, dass** man die Dispersion der Trägerschicht (c) hergestellt gemäss einem der Ansprüche 19 oder 20 auf die Deckschicht (a) aufträgt und anschliessend vom gegebenenfalls anwesenden organischen Lösungsmittel befreit bzw. trocknet; und auf die getrocknete Schicht (c) die abziehbare Schutzschicht (d) aufbringt.

24. Verfahren zur Herstellung des Pflasters nach Anspruch 1, **dadurch gekennzeichnet, dass** man die Dispersion der Trägerschicht (c), hergestellt gemäss einem der Ansprüche 20 oder 21 auf die abziehbare Schutzschicht (d) aufträgt und anschliessend vom gegebenenfalls anwesenden organischen Lösungsmittel befreit bzw. trocknet; in einem separaten Schritt die Bestandteile der Klebeschicht (b), in verflüssigtem Zustand oder gelöst in einem geeigneten organischen Lösungsmittel, auf die Deckschicht (a) aufträgt und anschliessend vom gegebenenfalls anwesenden organischen Lösungsmittel befreit bzw. trocknet; und anschliessend die Trägerschicht (c) mit der Deckschicht (a), welche bereits die Klebeschicht (b) enthält, kaschiert.

25. Verfahren zur Herstellung des Pflasters nach Anspruch 2, **dadurch gekennzeichnet, dass** man die Dispersion der Trägerschicht (c), hergestellt gemäss einem der Ansprüche 20 oder 21 auf die abziehbare Schutzschicht (d) aufträgt und anschliessend vom gegebenenfalls anwesenden organischen Lösungsmittel befreit bzw. trocknet; und auf die getrocknete Trägerschicht (c) die Deckschicht (a) aufbringt.

26. Verfahren nach einem der Ansprüche 22-25, **dadurch gekennzeichnet, dass** man die Dispersion der Trägerschicht (c) lösungsmittelfrei bei einer Temperatur im Bereich von 120°C-140°C, vorzugsweise im Bereich von 80°C-120°C und insbesondere auf etwa 100°C, aufträgt.

## Claims

1. A medical patch for administering to the skin the antiphlogistic agent etofenamate, which is liquid at room temperature, wherein said patch has a structure consisting of the top layer (a), the backing layer (c), the peelable protective layer (d) and optionally an intermediate layer (b), **characterized in that**:
- the top layer (a) consists of an inert material;
- the backing layer (c) consists of a self-adhesive polysiloxane as the backing material, which was obtained by condensing a dimethylpolysiloxane containing silanol groups with a silicate resin that is soluble in organic solvents and then reacting the remaining silanol groups with a reactive trimethylsilyl compound; and
- this self-adhesive polysiloxane optionally contains additives that are known *per se* for modifying the adhesive properties and optionally additives for reducing the viscosity of the self-adhesive polysiloxane, and into which the antiphlogistic agent is incorporated in the form of a dispersion, optionally with an agent that promotes permeation through the skin;
- the adhesive force of the backing layer (c) is in the range from 0.8 N/25 mm to 1.4 N/25 mm;
- the backing layer (c) contains the agent etofenamate in a concentration in the range from 1.0% to 25.0% by weight, based on the total weight of the backing layer (c);
- the backing layer (c) contains the agent in dispersed form in an average droplet size in the range from 0.1 µm to 500 µm;
- the backing layer (c) adheres directly to the top layer (a) or is optionally attached to it by the intermediate layer (b) ;
- the peelable protective layer (d) consists of an inert material, adheres to the backing layer (c) and can be easily peeled off of it.

2. The patch according to claim 1, which has a structure consisting of the top layer (a), the backing layer (c) and the peelable protective layer (d), **characterized in that**:
- the top layer (a) consists of an inert material;
- the backing layer (c) consists of a self-adhesive polysiloxane, in which the antiphlogistic agent is incorporated in the form of a dispersion, optionally with an agent that promotes permeation through the skin and optionally with further additives, and this backing layer (c) adheres directly to the top layer (a); and
- the peelable protective layer (d) consists of an inert material, adheres to the backing layer (c) and can be easily peeled off of it.

3. The patch according to claim 1, **characterized in that** an intermediate layer (b) is present between the top layer (a) and the backing layer (c), said adhesive layer (b) either not containing any of the agent or being loaded with a different amount of the agent, and the adhesive force of the adhesive layer (b) being stronger than the adhesive force of the backing layer (c), wherein the adhesive force of the backing layer (c) is only strong enough that the patch is not prevented from being easily and completely removed from the skin.

4. The patch according to claim 3, **characterized in that** the adhesive layer (b) is loaded with agent, and there is at least as much of the agent in the adhesive layer (b) as in the backing layer (c), wherein the adhesive layer (b) is preferably loaded to the saturation point with the agent.

5. The patch according to claims 1-4, **characterized in that** the top layer (a) consists of an elastic textile fabric, which is coated with a polymer material.

6. The patch according to claim 1 or claims 3-5, **characterized in that** the adhesive layer (b) consists of an organic polymer which is known *per se,* is preferably soluble in an organic solvent and has good adhesive properties, preferably selected from the group containing: 2-ethylhexyl acrylate, methyl acrylate, SBS polymers, styrene-butadiene-styrene block copolymers (SBS), styrene-butadiene block copolymers (SB) and mixtures of these copolymers with a glass transition temperature (Tg) of preferably lower than -22°C [T_{G}<(-22°C)], and these polymers optionally contain additives, preferably the glycerol ester of hydrogenated rosin or polyterpenes.

7. The patch according to claim 6, **characterized in that** the adhesive layer (b) consists primarily of 2-ethylhexyl acrylate and methyl acrylate, preferably of approximately 19.9% by weight 2-ethylhexyl acrylate and approximately 79.3% by weight methyl acrylate, with an average molecular weight in the range from 350,000 to 550,000 daltons, preferably approximately 400,000 to 500,000 daltons.

8. The patch according to claim 6, **characterized in that** the adhesive layer (b) consists of 17.0% by weight SB, 11.3% by weight SBS, 70.8% by weight glycerol ester of rosin and 0.9% by weight of an antioxidant.

9. The patch according to one of claims 1-8, **characterized in that** the backing layer (c) consists of a self-adhesive polysiloxane, which is selected from self-adhesive polysiloxanes registered as Dow Corning® BIO-PSA® 7-4201, Dow Corning® BIO-PSA 7-4560 and Dow Corning® BIO-PSA 7-4603.

10. The patch according to one of claims 1-9, **characterized in that** the additives for modifying the adhesive properties are selected from rosin compounds, preferably dehydrogenated or hydrogenated rosin, glycerol ester of rosin, terpene resins, polyterpene resins, of alpha- or beta-pinene or a mixture of these compounds, or of low-viscosity silicones or polysiloxanes having terminal silanol groups.

11. The patch according to claim 10, **characterized in that** the backing layer (c) consists of a self-adhesive polysiloxane which can be used without the addition of a solvent and can be applied to the substrate during the production of the patch.

12. The patch according to one of claims 1-11, **characterized in that** the self-adhesive polysiloxane of the backing layer (c) contains at least one compound for reducing the viscosity of the self-adhesive polysiloxane, said compound being selected from the group containing glycerol and/or an ester compound of a medium-chain fatty acid with a monovalent alcohol, preferably an ester of propyl alcohol, isopropyl alcohol, butyl alcohol or isopropyl alcohol with a (C₁₀-C₁₆) fatty acid, preferably with a medium-chain (C₁₂-C₁₆) fatty acid, preferably with lauric acid, myristic acid or palmitic acid, preferably isopropyl myristate, wherein preferably approximately 2-15% by weight, preferably approximately 5-10% by weight of said compounds is present, based on the overall weight of the backing layer (c).

13. The patch according to one of claims 1-11, **characterized in that** the self-adhesive polysiloxane of the backing layer (c) contains at least one compound for reducing the viscosity of the self-adhesive polysiloxane, said compound being selected from the group containing ester compounds of a medium-chain fatty acid with a multivalent alcohol, preferably mono-, di-, or triester of glycerol with medium-chain (C₁₀-C₁₆) fatty acids, preferably mono-, di-, or triester of glycerol with medium-chain (C₁₂-C₁₆) fatty acids and/or natural oils, preferably olive oil or castor oil, wherein preferably approximately 2-15% by weight, preferably approximately 5-10% by weight of said compounds is present, based on the overall weight of the backing layer (c).

14. The patch according to one of claims 1-11, **characterized in that** the self-adhesive polysiloxane of the backing layer (c) contains at least one compound for reducing the viscosity of the self-adhesive polysiloxane, said compound being selected from the group containing liquid paraffin, polyoxyethylene sorbitan fatty acid ester compounds, preferably polyoxyethylene sorbitan monostearate, polyoxyethylene sorbitan monooleate, polyethylene glycol, preferably polyethylene glycol 400, propylene glycol, polypropylene glycol, esters of multivalent acids with alcohols, preferably triethyl citrate, and mixtures of said compounds, wherein preferably approximately 2-15% by weight, preferably approximately 5-10% by weight of said compounds is present, based on the overall weight of the backing layer (c).

15. The patch according to one of claims 1-14, **characterized in that** the backing layer (c) contains at least one compound for promoting permeation through the skin and optionally additional stabilizers and fragrances.

16. The patch according to claim 15, **characterized in that** the compound that promotes permeation through the skin (permeation enhancer) is selected from naturally occurring substances, preferably from natural oils and fats or from fatty acids and higher fatty alcohols and esters thereof as well as from glycerol and mixtures of said compounds, wherein the weight ratio of agent:permeation enhancer is preferably in the range of 98:2 to 2:8, preferably in the range from 9:1 to 3:7, preferably about 1:2.

17. The patch according to one of claims 1-16, **characterized in that** the backing layer (c) contains the agent etofenamate in a concentration in the range from 2.0% by weight to 20% by weight and preferably 2.5% by weight to 15% by weight, preferably in a concentration of approximately 5% by weight to approximately 10% by weight, based on the overall eight of the backing layer (c).

18. The patch according to one of claims 1-17, **characterized in that** the backing layer (c) contains the agent in dispersed form with an average droplet size in the range from 1.0 µm to 100 µm.

19. The patch according to one of claims 1-18, **characterized in that** the coating of the surface for the backing layer (c) lies in the range from 30 g/m² to 300 g/m², preferably in the range from 40 g/m² to 200 g/m², preferably in the range from 40 g/m² to 100 g/m².

20. A method for preparing the dispersion that can be used as a backing layer (c) according to one of claims 1-19, **characterized in that** the self-adhesive polysiloxane that forms the backing layer (c) is mixed together with the agent, optionally with a substance that promotes permeation through the skin and optionally further additives, at a temperature in the range of 80°C-190°C, preferably in the range of 140°C-180°C and in particular in the range of 160°C-180°C, until the desired dispersion of the agent has formed in the matrix, and is subsequently allowed to cool to a temperature in the range of 120°C-140°C, preferably in the range of 80°C-120°C and in particular to approximately 100°C, the dispersion is applied to the desired substrate at this temperature in the form of a "hot melt" and is processed into the backing layer (c), and is optionally rid of any organic solvent that may still be present before or after lamination.

21. A method for preparing the dispersion that can be used as a backing layer (c) according to one of claims 1-19, **characterized in that** the self-adhesive polysiloxane that forms the backing layer (c) is heated together with the agent, optionally with a substance that promotes permeation through the skin and optionally further additives, in the presence of sufficient solvent at an elevated temperature, preferably in the range of 40°C-90°C, preferably in the range of the boiling point of the solvent, and stirred vigorously until the desired dispersion has formed, and the solvent is subsequently removed substantially or entirely from the formed dispersion.

22. A method for producing the patch according to claim 1, **characterized in that** the components of the adhesive layer (b) are applied to the top layer (a), either in liquefied form or dissolved in a suitable organic solvent, and subsequently rid of the organic solvent that may be present, respectively dried; the dispersion of the backing layer (c) prepared according to one of claims 19 and 20 is subsequently applied to the adhesive layer (b) and rid of any organic solvent that may be present and then dried; and the peelable protective layer (d) is applied to the dried backing layer (c).

23. A method for producing the patch according to claim 2, **characterized in that** the dispersion of the backing layer (c) prepared according to one of claims 19 and 20 is applied to the top layer (a) and then rid of any organic solvent that may be present, respectively dried; and the peelable protective layer (d) is applied to the dried layer (c).

24. A method for producing the patch according to claim 1, **characterized in that** the dispersion of the backing layer (c) prepared according to one of claims 20 and 21 is applied to the peelable protective layer (d) and subsequently rid of any organic solvent that may be present and then dried; in a separate step, the components of the adhesive layer (b) are applied to the top layer (a), either in liquefied form or dissolved in a suitable organic solvent, and subsequently rid of any organic solvent that may be present, respectively dried; and the backing layer (c) is then laminated with the top layer (a), which already contains the adhesive layer (b).

25. A method for producing the patch according to claim 2, **characterized in that** the dispersion of the backing layer (c) prepared according to one of claims 20 and 21 is applied to the peelable protective layer (d) and subsequently rid of any organic solvent that may be present, respectively dried; and the top layer (a) is applied to the dried backing layer (c).

26. The method according to one of claims 22-25, **characterized in that** the dispersion of the backing layer (c) is applied without solvent at a temperature in the range of 120°C-140°C, preferably in the range of 80°C-120°C and in particular at approximately 100°C.

## Revendications

1. Pansement médical pour le dépôt du principe actif étofénamate à effet antiphlogistique liquide à température ambiante sur la peau, dans lequel ce pansement présente une structure constituée de la couche supérieure (a), de la couche de support (c), de la couche protectrice détachable (d), et le cas échéant, d'une couche intermédiaire (b), **caractérisé en ce que** :
- la couche supérieure (a) est constituée d'un matériau inerte ;
- la couche de support (c) est constituée d'un polysiloxane autocollant faisant office de matériau de support, lequel a été obtenu par condensation d'un diméthylpolysiloxane contenant des groupes silanol avec une résine de silicate soluble dans des solvants organiques, puis par transformation des groupes silanols restants avec un composé de triméthylsilyl réactif ; et
- ce polysiloxane autocollant contient le cas échéant des additifs connus en soi pour la modification des propriétés adhésives, et le cas échéant des additifs pour diminuer la viscosité du polysiloxane autocollant, et dans lequel le principe actif à effet antiphlogistique est stocké sous la forme d'une dispersion, le cas échéant avec un agent favorisant la perméation à travers la peau ;
- le pouvoir adhésif de la couche de support (c) est compris dans la plage allant de 0,8 N/25 mm à 1,4 N/25 mm ;
- la couche de support (c) contient le principe actif étofénamate dans une concentration comprise dans la plage allant de 1,0 % en poids à 25,0 % en poids, calculé par rapport au poids total de la couche de support (c) ;
- la couche de support (c) contient le principe actif se présentant sous forme dispersée dans une taille des gouttelettes moyenne dans une plage allant de 0,1 µm à 500 µm;
- la couche de support (c) adhère directement à la couche supérieure (a), ou le cas échéant est reliée à celle-ci via la couche intermédiaire (b) ; et
- la couche protectrice détachable (d) est constituée d'un matériau inerte, adhère à la couche de support (c) et est facilement détachable de celle-ci.

2. Pansement selon la revendication 1, lequel présente une structure constituée de la couche supérieure (a), de la couche de support (c) et de la couche protectrice détachable (d), **caractérisé en ce que** :
- la couche supérieure (a) est constituée d'un matériau inerte,
- la couche de support (c) est constituée d'un polysiloxane autocollant, dans lequel le principe actif à effet antiphlogistique est stocké sous la forme d'une dispersion, le cas échéant avec un agent favorisant la perméation à travers la peau et le cas échéant d'autres additifs, stocké sous la forme d'une dispersion, et cette couche de support (c) adhère directement à la couche supérieure (a), et
- la couche protectrice détachable (d) est constituée d'un matériau inerte, adhère à la couche de support (c) et est facilement détachable de celle-ci.

3. Pansement selon la revendication 1, **caractérisé en ce qu'**une couche intermédiaire (b) est présente entre la couche supérieure (a) et la couche de support (c), cette couche adhésive (b) ne contient soit aucun principe actif, soit est chargée avec un principe actif en différentes quantités, et la puissance adhésive de la couche adhésive (b) est supérieure à la puissance adhésive de la couche de support (c), dans lequel la puissance adhésive de la couche de support (c) est uniquement suffisante pour que le pansement puisse être retiré de la peau facilement et intégralement.

4. Pansement selon la revendication 3, **caractérisé en ce que** la couche adhésive (b) est chargée en principe actif et le principe actif est présent dans au moins la même quantité dans la couche adhésive (b) que le principe actif dans la couche de support (c), dans lequel la couche adhésive (b) est de préférence chargée avec le principe actif jusqu'à la limite de saturation.

5. Pansement selon l'une quelconque des revendications 1-4, **caractérisé en ce que** la couche supérieure (a) est constituée d'une structure textile plane élastique, laquelle est revêtue d'un matériau polymère.

6. Pansement selon l'une quelconque des revendications 1, ou 3-5, **caractérisé en ce que** la couche supérieure (b) est constituée d'un polymère organique doté de bonnes propriétés adhésives connu en tant que tel, de préférence soluble dans un solvant organique, de préférence sélectionné parmi le groupe contenant : l'acrylate de 2-éthylhexyl, l'acrylate de méthyle, le polymère SBS, les copolymères en bloc styrène-butadiène-styrène (SBS), les copolymères en bloc styrène-butadiène (SB) et des mélanges de ces copolymères dotés d'une température de transition vitreuse (T_{G}) de préférence inférieure à -22 °C [T_{G}<(-22 °C)], et ces polymères contiennent le cas échéant des additifs, de préférence l'ester de glycérine du colophane hydraté ou des polyterpènes.

7. Pansement selon la revendication 6, **caractérisé en ce que** la couche adhésive (b) est essentiellement constituée d'acrylate de 2-éthylhexyl et d'acrylate de méthyle, de préférence d'environ 19,9 % en poids d'acrylate de 2-éthylhexyl et environ 73,9 % en poids d'acrylate de méthyle, doté d'un poids moléculaire moyen compris dans la plage allant de 350 000 à 550 000 dalton, de préférence environ 400 000 à 500 000 dalton.

8. Pansement selon la revendication 6, **caractérisé en ce que** la couche adhésive (b) est constituée à 17,0 % en poids de SB, 11,3 % en poids de SBS, 70,8 % en poids de l'ester glycérique de de colophane et de 0,9 % en poids d'un antioxydant.

9. Pansement selon l'une quelconque des revendications 1-8, **caractérisé en ce que** la couche de support (c) est constituée d'un polysiloxane autocollant, lequel est sélectionné parmi les polysiloxanes autocollants enregistrés sous les appellations Dow Corning® BIO-PSA® 7-4201, Dow Corning® BIO-PSA® 7-4560 et Dow Corning® BIO-PSA® 7-4603.

10. Pansement selon l'une quelconque des revendications 1-9, **caractérisé en ce que** les additifs pour la modification des propriétés adhésives sont sélectionnés parmi les composés de colophane, de préférence le colophane déshydraté ou hydraté, l'ester glycérique de colophane, les résines de terpène, les résines de polyterpène, parmi les alpha- ou bêta-pinènes ou un mélange de ces composés, ou parmi des silicones ou polysiloxanes à faible viscosité, lesquels possèdent des groupes silanol en position terminale.

11. Pansement selon la revendication 10, **caractérisé en ce que** la couche de support (c) est constituée d'un polysiloxane autocollant, lequel peut être utilisé lors de la production du pansement sans ajout d'un solvant, ou peut être appliqué sur le support.

12. Pansement selon l'une quelconque des revendications 1-11, **caractérisé en ce que** le polysiloxane autocollant de la couche de support (c) contient au moins un composé pour diminuer la viscosité du polysiloxane autocollant, lequel est sélectionné parmi le groupe contenant la glycérine et/ou un composé d'ester d'un acide gras à chaîne moyenne doté d'un alcool monovalent, de préférence un ester d'alcool propylique, d'alcool isopropylique, d'alcool butylique ou d'alcool isopropylique doté d'un acide gras en (C₁₀-C₁₆), de préférence doté d'un acide gras en (C₁₂-C₁₆) à chaîne moyenne, de préférence avec de l'acide dodécanoïque, de l'acide tétradécanoïque ou de l'acide palmitique, de préférence le myristate d'isopropyle, dans lequel est présent de préférence environ 2-15 % en poids, de préférence environ 5-10 % en poids de ces composés, rapporté au poids total de la couche de support (c).

13. Pansement selon l'une quelconque des revendications 1-11, **caractérisé en ce que** le polysiloxane autocollant de la couche de support (c) contient au moins un composé pour la diminution de la viscosité du polysiloxane autocollant, lequel est sélectionné parmi le groupe contenant des composés d'ester d'un acide gras à chaîne moyenne doté d'un alcool multivalent, de préférence un mono-, di- ou triester de glycérine doté d'acides gras en (C₁₀-C₁₆) à chaîne moyenne, de préférence un mono-, di- ou triester de glycérine doté d'acides gras en (C₁₂-C₁₆) à chaîne moyenne et/ou d'huiles naturelles, de préférence de l'huile d'olive ou de l'huile de ricin, lequel contient de préférence environ 2-15 % en poids, de préférence 5-10 % en poids de ces composés, rapporté au poids total de la couche de support (c).

14. Pansement selon l'une quelconque des revendications 1-11, **caractérisé en ce que** le polysiloxane autocollant de la couche de support (c) contient au moins un composé pour la diminution de la viscosité du polysiloxane autocollant, lequel est sélectionné parmi le groupe contenant la paraffine liquide, des composés d'ester d'acide gras polyoxyéthylène sorbitane, de préférence le monostéarate de polyoxyéthylène sorbitane, le monooléate de polyoxyéthylène sorbitane, le polyéthylène glycol, de préférence le polyéthylène glycol 400, le propylène glycol, le polypropylène glycol, l'ester d'acides multivalents dotés d'alcools, de préférence le citrate de triéthyle, et des mélanges de ces composés, lequel contient de préférence environ 2-15 % en poids, de préférence environ 5-10 % en poids de ces composés, rapporté au poids total de la couche de support (c).

15. Pansement selon l'une quelconque des revendications 1-14, **caractérisé en ce que** la couche de support (c) contient au moins un composé favorisant la perméation à travers la peau et le cas échéant des stabilisateurs et des substances aromatiques supplémentaires.

16. Pansement selon la revendication 15, **caractérisé en ce que** le composé favorisant la perméation à travers la peau (amplificateur de perméation ou
« permeation enhancer ») est sélectionné parmi les substances d'origine naturelle, de préférence parmi les huiles et matières grasses naturelles ou parmi les acides gras et alcools gras supérieurs et esters de ceux-ci ainsi que parmi la glycérine et les mélanges de ces composés, dans lequel le rapport de poids principe actif sur amplificateur de perméation est compris dans la plage allant de 98:2 à 2:8, de préférence dans la plage allant de 9:1 à 3:7, de préférence à environ 1:2.

17. Pansement selon l'une quelconque des revendications 1-16, **caractérisé en ce que** la couche de support (c) contient le principe actif étofénamate dans une concentration comprise dans une plage allant de 2,0 % en poids à 20 % en poids, et préférablement de 2,5 % en poids à 15 % en poids, de préférence dans une concentration d'environ 5 % en poids à environ 10 % en poids, calculé par rapport au poids total de la couche de support (c).

18. Pansement selon l'une quelconque des revendications 1-17, **caractérisé en ce que** la couche de support (c) contient le principe actif sous forme dispersée avec une taille de gouttelettes moyenne comprise dans une plage allant de 1,0 µm à 100 µm.

19. Pansement selon l'une quelconque des revendications 1-18, **caractérisé en ce que** le remplissage de la surface de la couche de support (c) est compris dans une plage allant de 30 g/m² à 300 g/m², de préférence dans une plage allant de 40 g/m² à 200 g/m², de préférence dans une plage allant de 40 g/m² à 100 g/m².

20. Procédé de production de la dispersion utilisable en tant que couche de support (c) selon l'une quelconque des revendications 1-19, **caractérisé en ce que** l'on mélange le polysiloxane autocollant formant la couche de support (c) avec le principe actif, le cas échéant avec un agent favorisant la perméation à travers la peau et le cas échéant d'autres additifs, à une température comprise dans une plage allant de 80 °C-190 °C, de préférence dans une plage allant de 140 °C-180 °C et notamment dans une plage allant de 160 °C-190 °C aussi longtemps que nécessaire, jusqu'à ce que la dispersion souhaitée du principe actif se forme dans la matrice, que l'on laisse ensuite refroidir à une température comprise dans une plage allant de 120 °C-140 °C, de préférence dans une plage allant de 80 °C-120 °C et notamment à environ 100 °C, que l'on applique la dispersion à cette température sous la forme d'un « hot-melt » sur le support souhaité et que l'on traite pour la couche de support (c), et que l'on libère le cas échéant avant ou après le laminage, du solvant organique encore présent le cas échéant.

21. Procédé de production de la dispersion utilisable en tant que couche de support (c) selon l'une quelconque des revendications 1-19, **caractérisé en ce que** l'on chauffe aussi longtemps que nécessaire le polysiloxane autocollant formant la couche de support (c) avec le principe actif, le cas échéant avec un agent favorisant la perméation à travers la peau et le cas échéant d'autres additifs, en présence de suffisamment de solvant à température élevée, de préférence dans une plage allant de 40 °C-90 °C, de préférence dans une plage du point d'ébullition du solvant, par un mélange intensif, jusqu'à ce que la dispersion souhaitée se soit formée, et que l'on élimine ensuite dans une large partie ou complètement le solvant de la dispersion formée.

22. Procédé de production du pansement selon la revendication 1, **caractérisé en ce que** l'on applique les composants de la couche adhésive (b), en état condensé ou dissous dans un solvant organique adéquat, sur la couche supérieure (a) et l'on libère ou sèche ensuite du solvant organique présent le cas échéant ; puis que l'on applique la dispersion de la couche de support (c) produite conformément à l'une quelconque des revendications 19 ou 20 sur la couche adhésive (b), puis l'on libère ou sèche du solvant organique présent le cas échéant ; et que l'on applique la couche protectrice détachable (d) sur la couche de support sèche (c).

23. Procédé de production du pansement selon la revendication 2, **caractérisé en ce que** l'on applique la dispersion de la couche de support (c) produite conformément à l'une quelconque des revendications 19 ou 20 sur la couche supérieure (a) et que l'on libère ou sèche ensuite le solvant organique présent le cas échéant ; et que l'on applique la couche protectrice détachable (d) sur la couche séchée (c).

24. Procédé de production du pansement selon la revendication 1, **caractérisé en ce que** l'on applique la dispersion de la couche de support (c), produite conformément à l'une quelconque des revendications 20 ou 21 sur la couche protectrice (d) détachable et que l'on libère ou sèche ensuite le solvant organique présent le cas échéant ; dans une étape séparée, que l'on applique les composants de la couche adhésive (b), dans un état condensé ou dissous dans un solvant organique adéquat, sur la couche supérieure (a), et que l'on libère ou sèche ensuite du solvant organique présent le cas échéant ; puis l'on dissimule la couche de support (c) avec la couche supérieure, laquelle contient déjà la couche adhésive (b).

25. Procédé de production du pansement selon la revendication 2, **caractérisé en ce que** l'on applique la dispersion de la couche de support (c), fabriquée conformément à l'une quelconque des revendications 20 ou 21, sur la couche protectrice détachable (d) puis que l'on libère ou sèche le solvant organique présent le cas échéant ; et que l'on applique la couche supérieure (a) sur la couche de support (c) sèche.

26. Procédé selon l'une quelconque des revendications 22-25, **caractérisé en ce que** l'on applique la dispersion de la couche de support (c) exempte de solvant à une température comprise dans une plage allant de 120 °C-140 °C, de préférence dans une plage allant de 80 °C-120 °C et notamment à environ 100 °C.
